# EUROPEAN PATENT SPECIFICATION

(11) **EP 2 614 857 B1**
(45) Date of publication and mention of the grant of the patent: **03.06.2020**
(21) Application number: 11823356.8
(22) Date of filing: 29.07.2011
(51) Int. Cl.: A61N 1/44, B62B 9/10, B62B 9/14, H01T 23/00

(54) **DEVICE FOR PREVENTION OR TREATMENT OF ATOPIC DERMATITIS**
VORRICHTUNG ZUR VORBEUGUNG ODER BEHANDLUNG ATOPISCHER EKZEME
DISPOSITIF POUR LA PRÉVENTION OU LE TRAITEMENT DE LA DERMATITE ATOPIQUE

(30) Priority: 11.07.2011 JP 2011152728; 06.09.2010 JP 2010198998
(43) Date of publication of application: 17.07.2013
(73) Proprietor: Sharp Kabushiki Kaisha, Osaka 590-8522 (JP)
(72) Inventor: OKANO, Hiroaki, Sakai City, Osaka 590-8522 (JP)
(74) Representative: Treeby, Philip David William
(86) International application number: PCT/JP2011/067442
(87) International publication number: WO 2012/032877

(56) References cited:
- WO-A1-02/32332
- WO-A1-96/32082
- JP-A- 6 000 210
- JP-A- 2004 321 535
- JP-A- 2006 026 022
- JP-A- 2009 189 785
- JP-A- 2010 075 661
- JP-A- 2010 137 047

## Description

### TECHNICAL FIELD

The present invention relates to a method for preventing or treating crisis of atopic dermatitis and a device for preventing or treating crisis of atopic dermatitis.

### BACKGROUND ART

The number of patients suffering from chronic atopic dermatitis has recently abruptly increased. A symptom of atopic dermatitis is represented by eczema with strong itching. At present, a method for treating atopic dermatitis is mostly represented by use of such a drug as external steroid or antihistamine. Such treatment temporarily ameliorates the symptom, however, there are many cases that, as soon as the treatment is stopped, the symptom becomes worse than before. Therefore, the drug should continually be administered, and in addition, in the case of the external steroid, administration per se for a long term has been difficult because of its side effects from prolonged administration.

Since atopic dermatitis is one type of allergic dermatitis, removal of an antigenic substance in an indoor space is also considered as one of methods for preventing or treating atopic dermatitis. Japanese Patent Laying-Open No. 2009-028681 (PTL 1) discloses an air cleaner for taking in such antigenic substances (allergen) as house dust and trapping the same with a filter. In addition, Japanese Patent Laying-Open No. 2004-268014 (PTL 2) discloses a method of deactivating an antigenic substance floating in the air by using positive ions and negative ions.

Atopic dermatitis, however, is also caused by a factor other than an environmental factor, such as a genetic factor. Therefore, though there may be a case that a symptom of atopic dermatitis is effectively mitigated by removing or deactivating an antigenic substance in an indoor space, such an effect is not absolute.

### CITATION LIST

### PATENT LITERATURE

PTL 1: Japanese Patent Laying-Open No. 2009-028681
PTL 2: Japanese Patent Laying-Open No. 2004-268014
PTL 3: Japanese Patent Laying-Open No. 2006-075358

A device according to the preamble of claim 1 is known from JP2010137047.

### SUMMARY OF INVENTION

The invention is defined in the appended claims. Any methods disclosed hereinafter do not form part of the scope of the invention.

### TECHNICAL PROBLEM

As described above, in the method for treating atopic dermatitis by using such a drug as external steroid or antihistamine, the drug should continually be administered. In addition, since the external steroid has a side effect caused by prolonged administration, administration per se for a long term is difficult. The antihistamine also causes such a side effect as arrhythmia, depending on a patient.

The present invention was made to solve the problems above, and an object thereof is to provide a device for preventing or treating crisis of atopic dermatitis without using a drug.

### SOLUTION TO PROBLEM

A method according to the present disclosure is characterized by preventing or treating atopic dermatitis of a patient by covering the patient itself or a patient's area affected by atopic dermatitis, filling a covered space with positive ions composed of H⁺(H₂O)ₘ (m being any integer) and negative ions composed of O₂⁻(H₂O)ₙ (n being any integer) which are generated as a result of discharge in an atmosphere, and bringing the positive ions and the negative ions into contact with a skin surface of the patient.

In the method according to the present disclosure, preferably, respective concentrations of the positive ions and the negative ions generated as a result of discharge are not less than 200000/cm³.

A medical device according to the present is characterized by preventing or treating atopic dermatitis by covering a patient itself or an affected area, filling a covered space with positive ions composed of H⁺(H₂O)ₘ (m being any integer) and negative ions composed of O₂⁻(H₂O)ₙ (n being any integer) which are generated as a result of discharge in an atmosphere, and bringing the positive ions and the negative ions into contact with a skin surface.

In the medical device according to the present invention, respective concentrations of the positive ions and the negative ions generated as a result of discharge are not less than 200000/cm³.

The medical device according to the present invention preferably includes a casing provided with a cavity for passage of an arm or a leg of a human therethrough and an ion generating element provided in the casing, for blowing ions into the cavity, the casing has an opening communicating with the cavity at each of opposing ends, and the opening has a sealing member for filling a gap between the arm or the leg of the human and the opening.

In the medical device according to the present invention, preferably, the casing has a bendable portion between the openings at the respective opposing ends.

In the medical device according to the present invention described above, the patient may be a non-human mammal or a human.

The present disclosure also provides a hooded stroller including a hood and an ion generating element within the hood, and the hooded stroller serves for preventing or treating atopic dermatitis by filling a space covered with the hood with positive ions composed of H⁺(H₂O)ₘ (m being any integer) and negative ions composed of O₂⁻(H₂O)ₙ (n being any integer) which are generated by the ion generating element and bringing the positive ions and the negative ions into contact with a skin surface of a baby or a toddler in the stroller.

### ADVANTAGEOUS EFFECTS OF INVENTION

According to the present invention, crisis of atopic dermatitis can be prevented or treated without using a drug.

### BRIEF DESCRIPTION OF DRAWINGS

Fig. 1 is a diagram schematically showing a device 1 for preventing or treating atopic dermatitis representing one preferred example of the present invention.
Fig. 2 is a cross-sectional view in a case where device 1 shown in Fig. 1 is applied to a human.
Fig. 3 is a diagram schematically showing a device 11 for preventing or treating atopic dermatitis representing another preferred example of the present invention.
Fig. 4 is a cross-sectional view of device 11 shown in Fig. 3.
Fig. 5 is a diagram schematically showing a case where device 11 shown in Fig. 3 is attached to an arm of a human.
Fig. 6 is a diagram schematically showing one example of an ion generating element suitably employed in the present invention.
Fig. 7 is a diagram schematically showing a hooded stroller 31 for preventing or treating atopic dermatitis representing one preferred example of the present invention.
Fig. 8 is a diagram schematically showing arrangement of a rearing cage placed in a testing room in Experimental Example 1, with (a) showing a PCI installation group and (b) showing an air cleaner installation group.
Fig. 9 shows a photograph of a cutaneous symptom of mice in group 1, group 3, and group 5 in Experimental Example 1.
Fig. 10 shows a photograph of a cutaneous symptom of mice in group 2, group 4, and group 6 in Experimental Example 1.
Fig. 11 shows a graph of an atopy score of the mice in group 2, group 4, and group 6.
Fig. 12 shows a graph of results of analysis with ELISA, of IgE in plasma in each group after the test ended in Experimental Example 1.
Fig. 13 shows a graph of results of analysis with ELISA, of IFN-γ in plasma in each group after the test ended in Experimental Example 1.
Fig. 14 shows a graph of results of analysis with ELISA, of IL-10 in plasma in each group after the test ended in Experimental Example 1.
Fig. 15 shows a photograph of inflammatory cells in each group at the time when the test ended in Experimental Example 1.
Fig. 16 shows a graph of results in Experimental Example 2, showing an average of a survival rate in each of a PCI installation group and a no-installation group.
Fig. 17 shows a graph of results in Experimental Example 2, showing a survival rate in each group resulting from grouping based on a degree of cutaneous reaction in the PCI installation group and the no-installation group.
Fig. 18 shows a graph of results in Experimental Example 2, showing a total average of the atopy score for each initial atopic symptom in the PCI installation group and the no-installation group.
Fig. 19 shows a graph of results in Experimental Example 2, showing an atopy score for each initial atopic symptom in the PCI installation group and the no-installation group.

### DESCRIPTION OF EMBODIMENTS

### <Method for Preventing or Treating Atopic Dermatitis>

The method for preventing or treating atopic dermatitis according to the present disclosure prevents or treats atopic dermatitis by covering a patient itself or a patient's area affected by atopic dermatitis, filling a covered space with positive ions composed of H⁺(H₂O)ₘ (m being any integer) and negative ions composed of O₂⁻(H₂O)ₙ (n being any integer) which are generated as a result of discharge in an atmosphere, and bringing the positive ions and the negative ions into contact with a skin surface of the patient.

Here, Japanese Patent Laying-Open No. 2006-075358 (PTL 3) describes a sterilization method characterized by releasing positive ions and negative ions onto a damaged, affected area or mucosal area of an animal and fragmenting proteins contained in microorganisms or viruses on condition that nucleic acids contained in the microorganisms or viruses present in the affected area or mucosal area are not disrupted. The method disclosed in this PTL 3 sterilizes microorganisms or viruses attached to a skin or to a mucosal area, and does not directly act on an animal's skin.

In contrast, the method according to the present disclosure does not prevent or treat atopic dermatitis by sterilizing microorganisms or viruses attached to a skin but prevents or treats atopic dermatitis by causing high-concentration positive ions and negative ions to directly act on the skin. Thus, the method according to the present invention is completely different in technical concept from the method disclosed in PTL 3.

A patient of interest of treatment or prevention in the method according to the present disclosure may be any of a human and a mammal other than human (a non-human mammal). Examples of the non-human mammals include dogs, bovine, cats, pigs, monkeys, rabbits, and rats. In addition, examples of areas affected by atopic dermatitis include skins of a hand and a leg (limbs), a body, and a face, and a scalp.

Positive ions composed H⁺(H₂O)ₘ (m being any integer) and negative ions O₂⁻(H₂O)ₙ (n being any integer) can be generated, for example, in such a manner that molecules of oxygen (O₂), water (H₂O), and the like in air receive energy through a discharge phenomenon at an ion generating element, and usually, positive ions and negative ions can simultaneously be generated and emitted into air by alternately applying positive and negative voltages. The method for generating positive ions and negative ions employed in the present disclosure, however, is not limited thereto, and only any of a positive voltage and a negative voltage can be applied to generate only any of positive ions and negative ions, and thereafter a reverse voltage can be applied to generate ions charged oppositely to already emitted ions. It is noted that an applied voltage necessary for generating these positive ions and negative ions is preferably in a range from 1.1 kV to 2.0 kV, although depending on a structure of an electrode.

With regard to composition of positive ions and negative ions generated through the discharge phenomenon with oxygen molecules and/or water molecules present on a surface of a discharge element serving as source materials, mainly as positive ions, water molecules in air are electrolytically dissociated through plasma discharge to generate hydrogen ions H⁺, which cluster together with water molecules in air owing to solvation energy to thereby form H⁺(H₂O)ₘ (m being any integer). Meanwhile, with regard to negative ions, oxygen molecules or water molecules in air are electrolytically dissociated through plasma discharge to generate oxygen ions O₂⁻, which cluster together with water molecules in air owing to solvation energy to thereby form O₂⁻(H₂O)ₙ (n being any integer). In addition, as a result of reaction between both of them, more active active species such as hydrogen peroxide H₂O₂, hydrogen dioxide O₂H, or hydroxyl radicals •OH can readily be generated.

In the present invention, respective concentrations of the positive ions and the negative ions generated as a result of discharge are not less than 200000/cm³. This is because, in the case where respective concentrations of the positive ions and the negative ions are less than 200000/cm³, an effect of prevention and treatment of atopy tends to be low. It is noted that, with regard to definition of the number of ions, small ions were counted and critical mobility in air was assumed as 1 cm²/V•second.

Whether or not air contains such positive ions and negative ions can be determined by examining gas composition through gas mass spectroscopy, a gas concentration test, a color change test, an odor test, a light emission test, a generated sound test, or the like. Gas mass spectroscopy can make use of a conventionally known mass spectroscope, and in a gas concentration test, measurement can be conducted by making use of gas chromatography or an ion counter. In a color change test or an odor test, a sensory test such as visual inspection or olfactometry can be conducted, and in addition, a color difference meter, an odor sensor, or the like can also be made use of. Moreover, in a light emission test or a generated sound test, a sensory test such as visual inspection or an auditory test can again be conducted, and in addition, an absorptiometer, a spectroscope, an optical sensor, an illuminometer, a microphone, or the like can be made use of.

### <Medical Device for Preventing or Treating Atopic Dermatitis>

The present invention also provides a medical device for preventing or treating atopic dermatitis by covering a patient itself or an affected area, filling a covered space with positive ions composed of H⁺(H₂O)ₘ (m being any integer) and negative ions composed of O₂⁻(H₂O)ₙ (n being any integer) which are generated as a result of discharge in an atmosphere, and bringing the positive ions and the negative ions into contact with a skin surface of the patient. Details of the device according to the present invention are also the same as described above in connection with the method according to the present invention, and respective concentrations of the positive ions and the negative ions generated as a result of discharge are not less than 200000/cm³. As in the method according to the present described above, a patient of interest of prevention or treatment by the medical device according to the present invention may be any of a human and a non-human mammal.

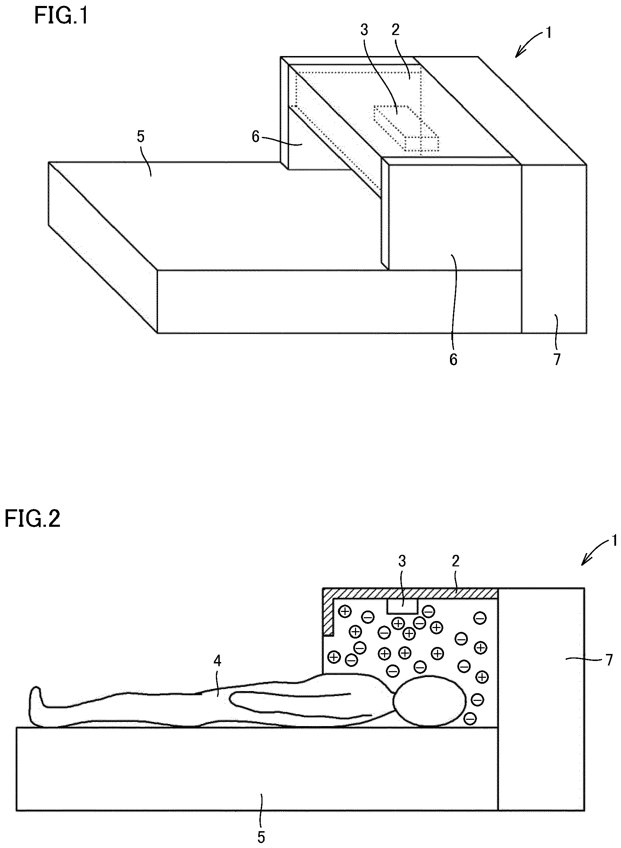

Fig. 1 is a diagram schematically showing a device 1 for preventing or treating atopic dermatitis representing one preferred example of the present invention and Fig. 2 is a cross-sectional view thereof in a case where it is applied to a human (patient) 4. In device 1 in the example shown in Figs. 1 and 2, a side panel 6 is placed at an end portion on each of opposing sides where a head side of human 4 lying on a bed 5 is located, and a cover 2 is placed as being fixed to each side panel 6 such that the cover is located above the head when the human lies on bed 5. A vertical distance between cover 2 and bed 5 is set such that a gap sufficient for human 4 lying on bed 5 to be able to turn over is provided. In addition, a head-side side panel 7 is provided at an end portion in a longitudinal direction on the head side of bed 5 when human 4 lies thereon, so that a space covered with cover 2, side panels 6, and head-side side panel 7 is formed.

In the example shown in Figs. 1 and 2, an ion generating element 3 is placed on a space side of cover 2, and by driving this ion generating element 3, the space can be filled with positive ions composed of H⁺(H₂O)ₘ (m being any integer) and negative ions O₂⁻(H₂O)ₙ (n being any integer) at high concentration. In the example shown in Fig. 2, such device 1 can cover a human lying on bed 5 from his/her head portion approximately to a chest portion. Thus, by using device 1 during sleeping, atopic dermatitis in the head portion, a neck portion, or the like can be prevented or treated.

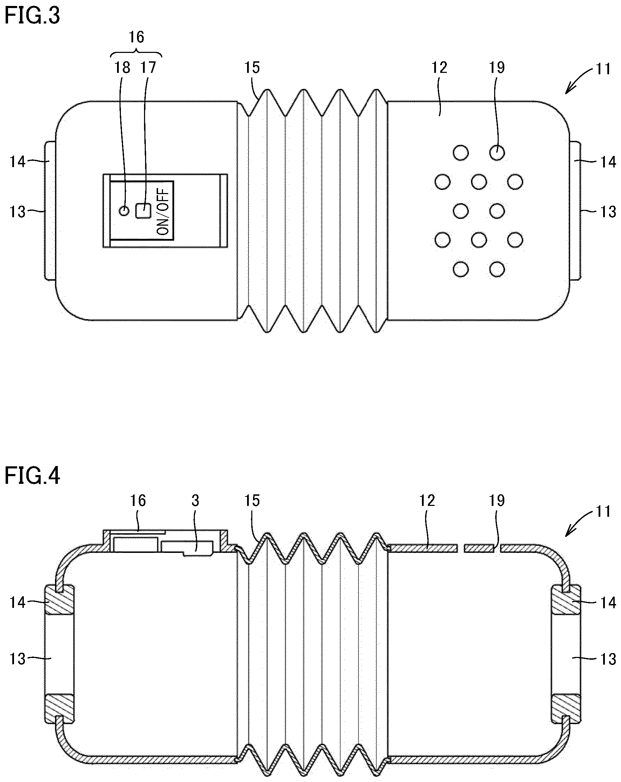

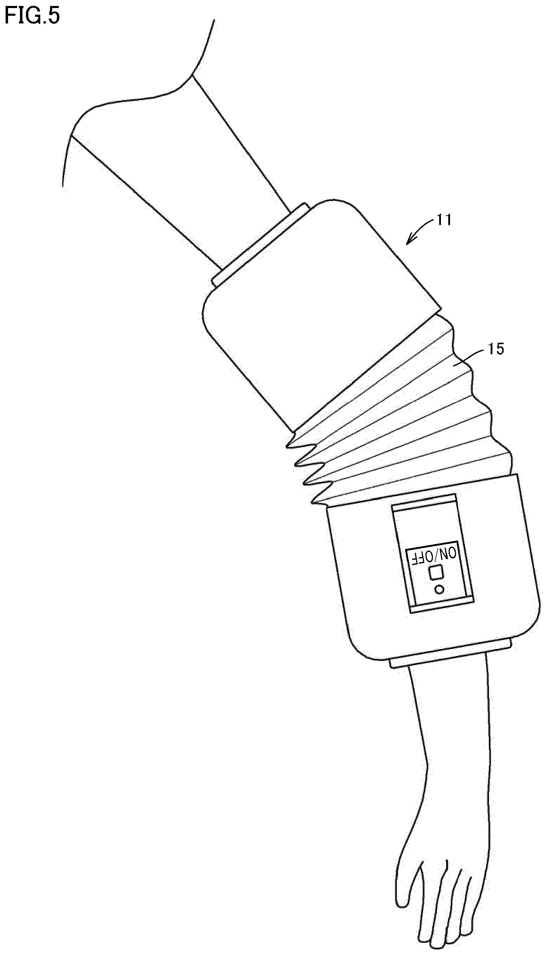

Fig. 3 is a diagram schematically showing a device 11 for preventing or treating atopic dermatitis representing another preferred example of the present invention and Fig. 4 is a cross-sectional view thereof. In addition, Fig. 5 is a diagram schematically showing a case where device 11 is attached to an arm of a human. The present invention also provides a device characterized by including a casing provided with a cavity for passage of an arm or a leg of a human therethrough and an ion generating element provided in the casing, for blowing ions into the cavity, the casing having an opening communicating with the cavity at each of opposing ends, and the opening having a sealing member for filling a gap between the arm or the leg of the human and the opening.

In the example shown in Figs. 3 to 5, a casing 12 has a substantially cylindrical shape and has an opening 13 at each of opposing ends thereof. Opening 13 is provided with a ring-shaped sealing member 14. Sealing member 14 is made of urethane having cushioning properties, which is externally covered with cloth (not shown). Sealing member 14 can be replaced with one having a size optimal to an arm or a leg of a user. It is noted that sealing member 14 does not necessarily have to completely fill a gap, and a slight gap may be acceptable so long as a space between an arm or a leg and the opening is approximately covered.

As in the example shown in Figs. 3 to 5, casing 12 preferably includes a portion which can be bent (a bendable portion) 15 between openings 13 at the opposing ends. Bendable portion 15 is formed, for example, from a bellows pipe made of urethane, and one end of casing 12 can be bent with respect to the other end. An operation portion 16 is provided on a surface of casing 12. Operation portion 16 is provided, for example, with a switch 17 for turning ON/OFF power and an LED 18 for checking a power ON/OFF state (for example, which is turned on in a power ON state and turned off in a power OFF state).

In addition, ion generating element 3 is provided in casing 12 so as to be able to emit ions into a cavity in the inside of casing 12. A drive circuit for controlling drive of ion generating element 3 is provided in the vicinity of ion generating element 3. The drive circuit is connected to operation portion 16 and ion generating element 3, and it is implemented to be able to control drive of ion generating element 3 through operation of switch 17 in operation portion 16.

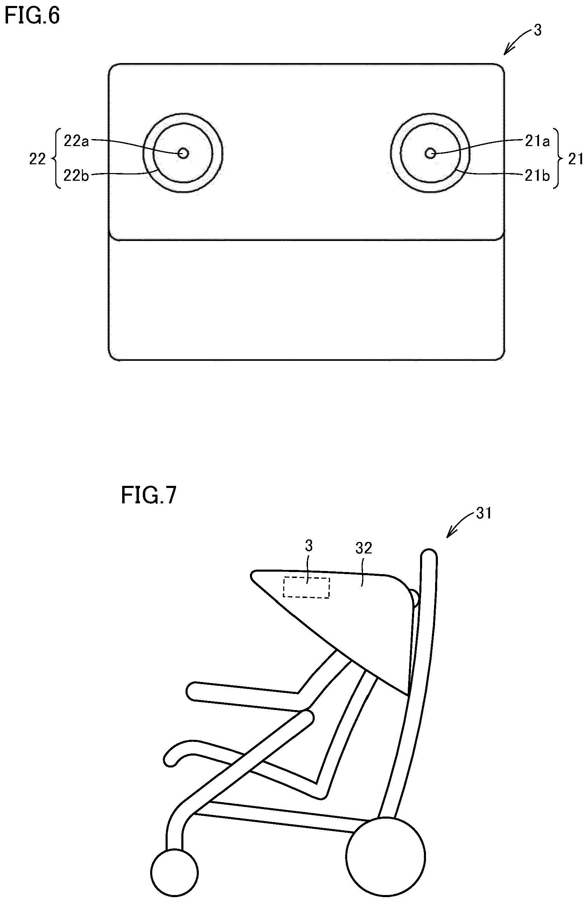

Here, Fig. 6 is a diagram schematically showing one example of ion generating element 3 suitably employed in the present invention. Ion generating element 3 holds two ion generating portions 21, 22 with a holder, for example, as in the example shown in Fig. 6. A power feed portion for supplying a voltage to ion generating portions 21, 22 is provided in the drive circuit. As the voltage is supplied from the power feed portion to ion generating portions 21, 22, ion generating portions 21, 22 generate ions.

Ion generating portions 21, 22 have needle-shaped discharge electrodes 21a, 22a and induction electrode rings 21b, 22b surrounding discharge electrodes 21a, 22a, respectively. Discharge electrodes 21a, 22a are arranged in central portions of induction electrode rings 21b, 22b, respectively. As described below, one ion generating portion 21 is configured to generate positive ions and the other ion generating portion 22 is configured to generate negative ions. Polarity of ions generated from ion generating portions 21, 22 may be switched every prescribed time.

A positive voltage is applied to one ion generating portion 21, so that water molecules in air are electrolyzed in a plasma region resulting from discharge to thereby mainly generate hydrogen ions H⁺. Then, water molecules in air aggregate around generated hydrogen ions so that positive cluster ions H⁺(H₂O)ₘ (m being any integer) are formed. A negative voltage is applied to the other ion generating portion 22, so that oxygen molecules in air are electrolyzed in a plasma region resulting from discharge to thereby mainly generate oxygen ions O₂⁻. Then, water molecules in air aggregate around generated oxygen ions so that negative cluster ions O₂⁻(H₂O)ₙ (n being any integer) are formed.

In addition, device 11 in the example shown in Figs. 3 to 5 is provided with a small vent 19 connecting the cavity in casing 12 to the outside of casing 12. By providing vent 19 in casing 12, moisture in the cavity can escape during a period in which an amount of perspiration is great, such as during summer.

In using device 11, as shown in Fig. 5, an arm passes through openings 13 at the opposing ends of casing 12 and bendable portion 15 formed from the bellows pipe is set at a position of an elbow. As the bellows pipe of casing 12 is bent, a user can freely move an arm joint even in such a state that device 11 is attached to his/her arm. In addition, since the bellows pipe can freely extend and contract, attachment to an arm is easy.

Though Fig. 5 shows an example of attachment to an arm, attachment also to a leg is possible. In this case, such attachment that the bellows pipe is set at a knee joint portion will allow movement of a knee joint with device 11 being attached.

In general, in many cases, atopic dermatitis develops in an inner elbow or behind a knee. Ion generation device 1 in the present example can be used for preventing or treating such atopic dermatitis as developing in the inner elbow or behind the knee.

As an arm or a leg passes through the cavity in device 11, a narrow shielded space is formed around the arm or the leg. By emitting positive ions composed of H⁺(H₂O)ₘ (m being any integer) and negative ions composed of O₂⁻(H₂O)ₙ (n being any integer) into this narrow shielded space, concentration of the positive ions and concentration of the negative ions become very high in the shielded space. Therefore, by attaching device 11 to an arm or a leg during sleeping or the like, prevention or treatment of atopic dermatitis can effectively be achieved.

It is noted that device 11 in the example shown in Figs. 3 to 5 is provided with small vent 19 in casing 12 and thus the cavity and the outside air are not completely shielded from each other. An amount of ions which escape through this vent 19 to the outside air, however, is small, and hence creation of a space filled with positive ions/negative ions at high concentration is not much affected.

As switch 17 is turned ON, LED 18 is turned on, ion generating element 3 is driven by the drive circuit, and positive ions and negative ions are emitted. Device 11 may naturally be driven by a battery (not shown) contained in casing 12, so that it can be used also in a daily life in a house or the like.

The present invention also provides a hooded stroller for preventing or treating atopic dermatitis. Fig. 7 is a diagram schematically showing a hooded stroller 31 for preventing or treating atopic dermatitis representing one preferred example of the present invention. As in the example shown in Fig. 7, hooded stroller 31 according to the present invention is a stroller including a hood 32 and ion generating element 3 in the inside of hood 32. Hooded stroller 31 can cover a toddler or a child with hood 32 when hood 32 is extended, and it prevents or treats atopic dermatitis by filling hood 32 with positive ions composed of H⁺(H₂O)ₘ (m being any integer) and negative ions composed of O₂⁻(H₂O)ₙ (n being any integer) by using ion generating element 3 placed in hood 32 and bringing the positive ions and the negative ions into contact with a skin surface of the toddler or the child in the hooded stroller.

Hooded stroller 31 may incorporate, for example, a chargeable battery as a power supply for driving ion generating element 3. Alternatively, it may be implemented to include a solar cell panel on the outside of hood 32. By doing so, the ion generating element can also be driven without separately placing a power supply.

Though the present invention will be described below in further detail with reference to experimental examples, the present invention is not limited thereto.

### <Experimental Example 1>

NC/Nga mice spontaneously developing atopic dermatitis were used to conduct a test below. The NC/Nga mice are species widely recognized as a model animal of atopic dermatitis. The symptom is represented by cutaneous inflammation accompanying itch, and it is characterized by noticeable increase in plasma IgE during crisis. In addition, the present mice develop atopic dermatitis owing to a mite antigen. Since no crisis of atopic dermatitis is seen in mice kept under SPF (Specific Pathogen Free) conditions, an SPF animal was used as a control group.

These mice were grouped such that an average value of weight is substantially the same, and each group was kept in an individual cage. In addition, a tail of a mouse was marked for individual identification in the group. Table 1 shows details of grouping.

**Table 1**

| Group | Group Name | SPF/Conventional | The Number of Samples |
|---|---|---|---|
| Group 1 | Non-Treated Group | SPF | 6 |
| Group 2 | Non-Treated Group | Conventional | 10 |
| Group 3 | Plasma Cluster Installation Group | SPF | 6 |
| Group 4 | Plasma Cluster Installation Group | Conventional | 10 |
| Group 5 | Air Cleaner Installation Group | SPF | 6 |
| Group 6 | Air Cleaner Installation Group | Conventional | 10 |

As shown in Table 1, grouping into group 1 to group 6 was made. Group 1, group 3, and group 5 each contained 6 SPF mice, and group 2, group 4, and group 6 contained 10 Conventional (normally kept) mice. Group 1 and group 2 were defined as no-installation groups for which neither of an air cleaner and an ion generator was installed. Group 3 and group 4 were defined as PCI (plasma cluster ion) installation groups for which an ion generator for generating positive ions composed of H⁺(H₂O)ₘ (m being any integer) and negative ions composed of O₂⁻(H₂O)ₙ (n being any integer) was installed. Group 5 and group 6 were defined as air cleaner installation groups for which an air cleaner was installed.

Mice in each of these grouped groups were transferred to a rearing cage placed in an individual testing room with 8 tatami mats (approximately 13.26 m²) and kept for 3 weeks. A temperature in the testing room was kept at 23±1°C and a humidity was kept at 50±10%, and mice were kept under such conditions as continuous ventilation and bright and dark cycles of 12 hours. Drinking water was automatically fed and feed ingestion was automatic (ordinary pellets for rodents from Oriental Yeast Co., Ltd.).

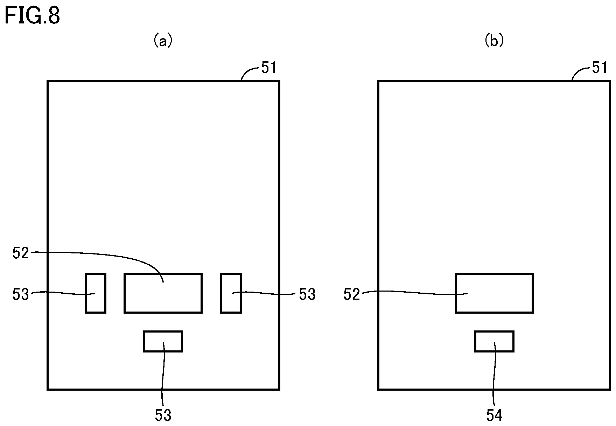

For the PCI installation group, as shown in Fig. 8 (a), three ion generating elements 53 (SHARP IG-B200) were installed in a testing room 51 and a plurality of ion generating elements were separately installed in a rearing cage 52, so that concentration of positive ions and concentration of negative ions in rearing cage 52 were adjusted to 200 thousand/cm³ (ion concentration was measured, with an inlet port of an ion counter being directed to a feeding portion of the cage and with an appropriate range being set).

For the air cleaner installation group, as shown in Fig. 8 (b), one air cleaner 54 (SHARP FU-Y53CX) was installed in testing room 51 and mice were kept in rearing cage 52 while air cleaner 54 was being operated. It is noted that the air cleaner was set not to generate ions. The air cleaner is provided with a HEPA filter and it can trap and remove an antigenic substance.

In each group, a cutaneous symptom of the mice was observed when the test was started (day 0), and on day 7, day 14, and day 21. The cutaneous symptom was determined by photographing and an atopy score (a degree of edema and erythema: 0=None; 1=Slight; 2=Moderate; 3=Severe).

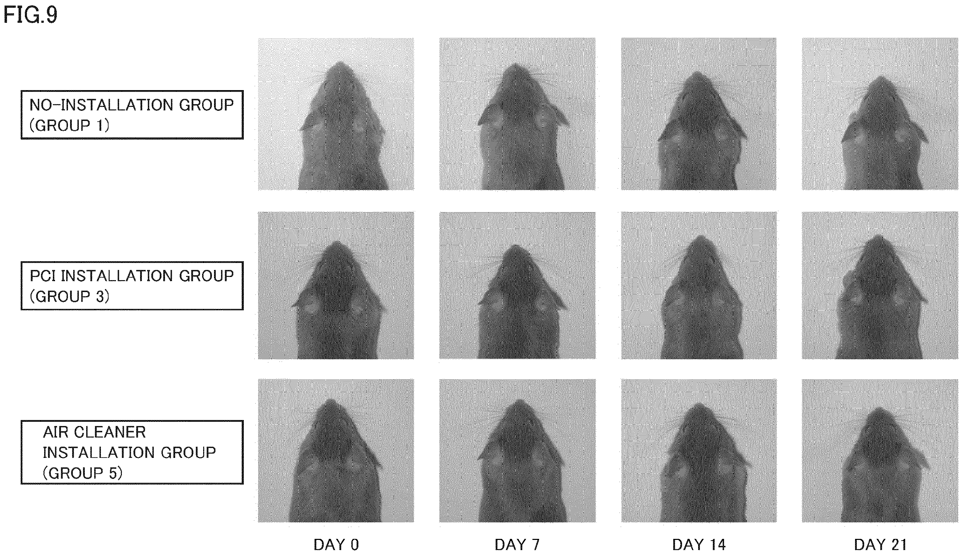

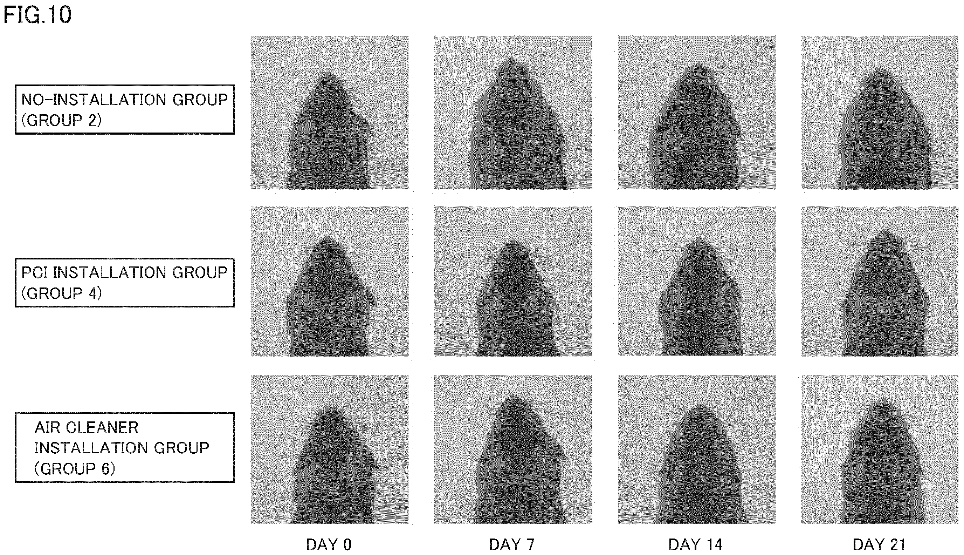

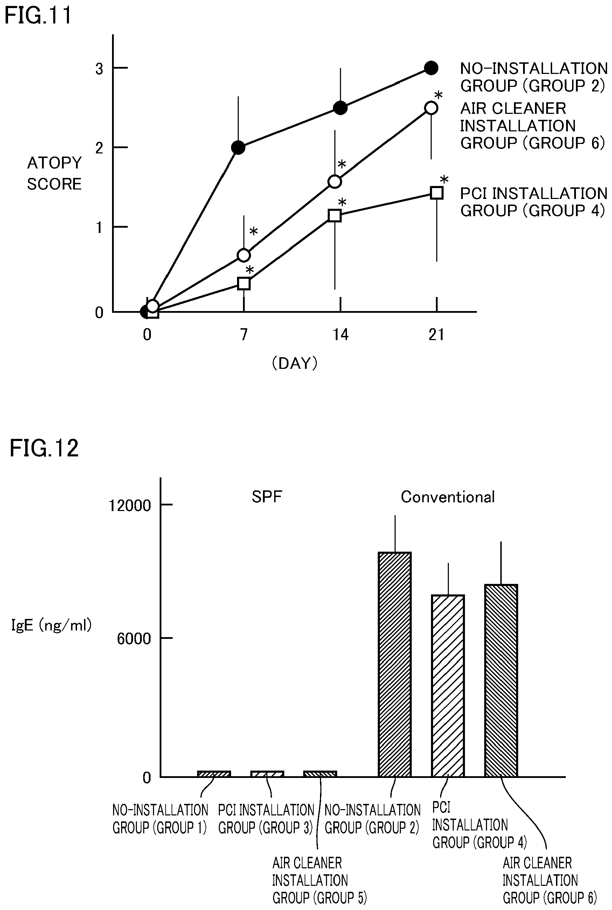

Fig. 9 shows a photograph of a cutaneous symptom of the mice in group 1, group 3, and group 5. In addition, Fig. 10 shows a photograph of a cutaneous symptom of the mice in group 2, group 4, and group 6. Fig. 11 shows an atopy score of the mice again in group 2, group 4, and group 6.

As shown in Fig. 9, no cutaneous reaction was found in the mice in group 1, group 3, and group 5 kept under the SPF conditions. On the other hand, as shown in Fig. 10, cutaneous reaction was found in the Conventional mice. Among them, the no-installation group exhibited the highest atopy score. The PCI installation group and the air cleaner installation group exhibited a significantly decreased atopy score as compared with the no-installation group. In addition, in comparison between the PCI installation group and the air cleaner installation group, the PCI installation group exhibited the atopy score significantly lower than that of the air cleaner installation group.

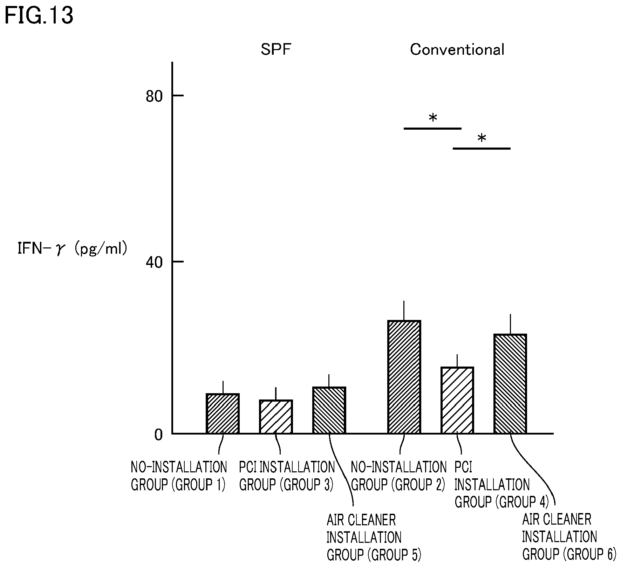

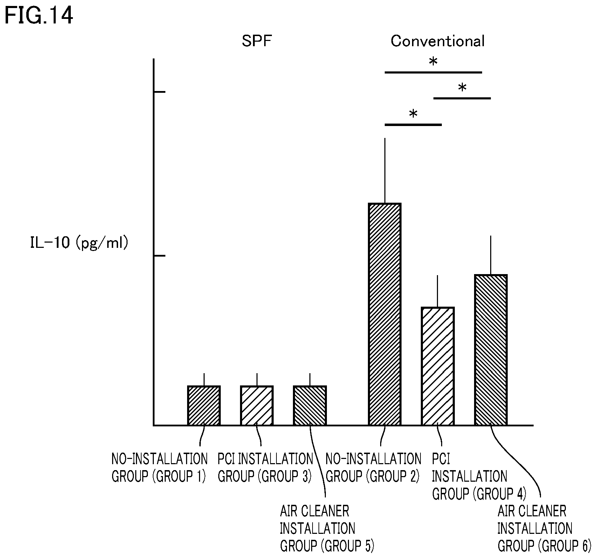

In addition, in each group, blood was taken at the time when the test ended (day 21) and plasma was separated. Thereafter, IgE, IFN-γ (interferon-gamma), and IL-10 in plasma were measured with ELISA. Figs. 12, 13, and 14 show the results, respectively.

In the Conventional mice, the content of IgE in plasma exhibited increase, however, no great difference was seen among the groups. In the Conventional mice, the content of IFN-γ and IL-10 in plasma exhibited increase, and in the PCI installation group and the air cleaner installation group, the content of IL-10 significantly decreased. Furthermore, in comparison between the PCI installation group and the air cleaner installation group, the PCI installation group exhibited a value significantly lower than that of the air cleaner installation group. The content of IFN-γ exhibited a significantly low value only in the PCI installation group.

It is noted that IgE, IFN-γ, and IL-10 in plasma and the atopy score were each shown as an average value ± a standard deviation. A test of significant difference was conducted in a t-test among the no-installation group, the PCI installation group, and the air cleaner installation group. It is noted that the test employed statistical analysis software (ANOVA) and a case where a level of significance was less than 5% was defined as significantly different.

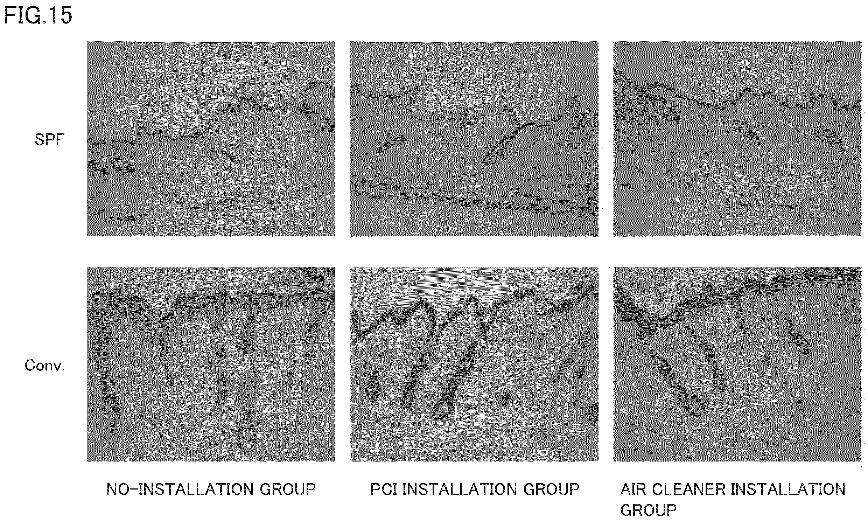

A dorsoanterior portion was taken when the test ended (day 21), HE stain was conducted, and a microscope was used to observe inflammatory cells. Fig. 15 shows the results. On day 21, significant thickening of a skin (in particular, epidermis) was observed in the Conventional no-installation group. In addition, edema and infiltration of inflammatory cells were observed. In the PCI installation group and the air cleaner installation group, as compared with the no-installation group, decrease in thickening of epidermis and infiltration of inflammatory cells were seen. In comparison between the PCI installation group and the air cleaner installation group, thickening of the epidermis was lighter in the PCI installation group than in the air cleaner installation group, and infiltration of inflammatory cells was also less.

From the foregoing, the mice in the PCI installation group in which the ion generator for generating positive ions composed of H⁺(H₂O)ₘ (m being any integer) and negative ions composed of O₂⁻(H₂O)ₙ (n being any integer) was installed were found to achieve effectively suppressed crisis of atopic dermatitis as compared with the mice in the air cleaner installation group where only the air cleaner not generating ions was installed. In spite of the fact that the PCI installation group and the air cleaner installation group can deactivate and remove an antigenic substance in air, there is a significant difference between the PCI installation group and the air cleaner installation group. Thus, it can be seen that positive ions composed of H⁺(H₂O)ₘ (m being any integer) and negative ions composed of O₂⁻(H₂O)ₙ (n being any integer) directly act on a living body itself and can suppress crisis of atopic dermatitis.

### <Experimental Example 2>

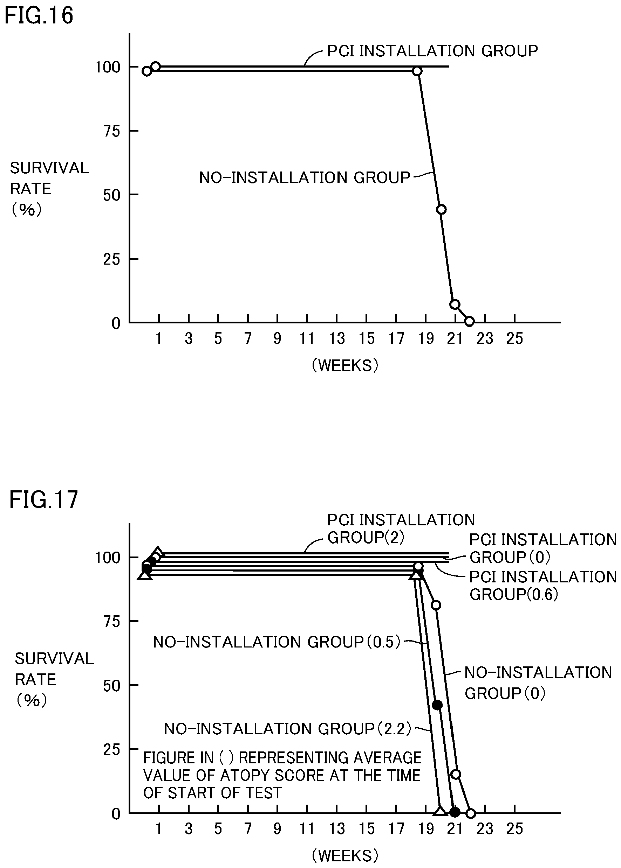

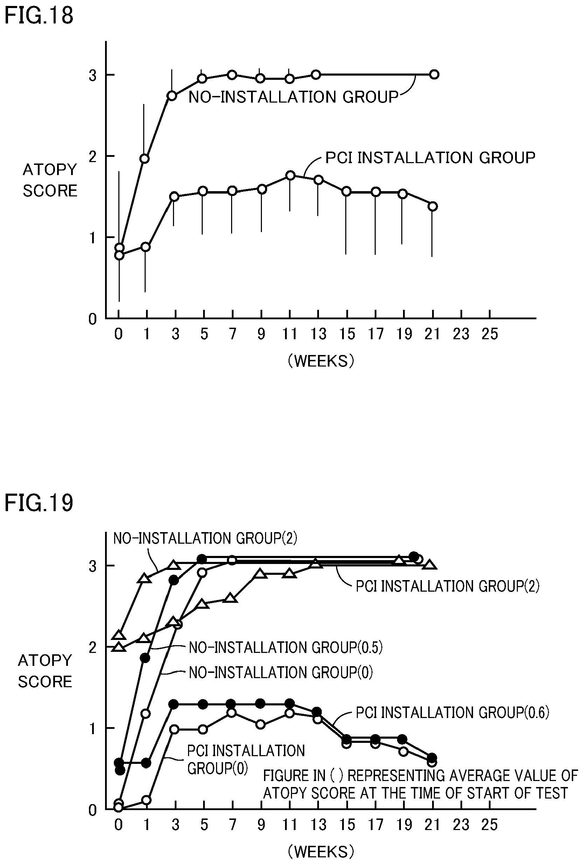

NC/Nga mice spontaneously developing atopic dermatitis were used, the mice were grouped into three based on a degree of cutaneous reaction in order to observe a treatment effect, each group was further grouped into two (a PCI installation group, a no-installation group) based on presence/absence of PCI, and a test was conducted in 6 groups in total. A degree of reaction was determined based on an atopy score (a degree of edema and erythema: 0=None; 1=Slight; 2=Moderate; 3=Severe) and an average was calculated. Figs. 16 and 17 show graphs of results in Experimental Example 2. Fig. 16 shows an average of a survival rate in each of the PCI installation group and the no-installation group and Fig. 17 shows a survival rate in each group resulting from grouping based on a degree of cutaneous reaction in the PCI installation group and the no-installation group. It is noted that, in Figs. 16 and 17, the ordinate represents a survival rate (%) and the abscissa represents weeks. In addition, Figs. 18 and 19 show graphs of results in Experimental Example 2. Fig. 18 shows a total average of an atopy score for each initial atopic symptom in the PCI installation group and the no-installation group and Fig. 19 shows an atopy score for each initial atopic symptom in the PCI installation group and the no-installation group. It is noted that a numeric value in parentheses (2, 0.6, 0.5, or 0) in the PCI installation group and the no-installation group shown in Figs. 17 and 19 show the atopy score above at the time point of start of the experiment. The atopy scores show as an initial atopic symptom of the mice, none with 0, slight with 0.5 (the no-installation group) or 0.6 (the PCI installation group), and severe with 2 (the PCI installation group, the no-installation group), respectively.

As shown in Fig. 17, the mice in the PCI installation group to which plasma cluster ions (positive ions composed of H⁺(H₂O)ₘ (m being any integer) and negative ions composed of O₂⁻(H₂O)ₙ (n being any integer)) were applied exhibited the survival rate of 100% regardless of a degree of atopy-like cutaneous reaction. In contrast, in the no-installation group, such a result was obtained that, after the 19th week since start of the test, all the mice died in the following 3 weeks.

In addition, as shown in Fig. 19, in the case where the initial atopic symptom was 2 indicating severe, even the PCI installation group to which the plasma cluster ions were applied exhibited 3 which is the highest value for the atopy score in the 15th week since start of the test. On the other hand, in the case where the initial atopic symptom was 0.6 indicating slight, in the PCI installation group to which the plasma cluster ions were applied, the atopy score was slightly higher than 1 from the 5th week to the 13th week since start of the test, whereas a tendency of gradual amelioration from the 17th week is seen. Furthermore, in the PCI installation group where the plasma cluster ions were applied even to asymptomatic mice, it can be seen that the atopy score temporarily increases, however, a tendency of amelioration is observed.

From Experimental Example 2 above, it was found that atopic dermatitis of mice could be treated in a space filled with plasma cluster ions (positive ions composed of H⁺(H₂O)ₘ (m being any integer) and negative ions composed of O₂⁻(H₂O)ₙ (n being any integer)).

It should be understood that the embodiments and the examples disclosed herein are illustrative and non-restrictive in every respect. The scope of the present invention is defined by the terms of the claims, rather than the description above, and is intended to include any modifications within the scope and meaning equivalent to the terms of the claims.

### REFERENCE SIGNS LIST

1, 11 device for preventing or treating atopic dermatitis; 2 cover; 3 ion generating element; 4 human; 5 bed; 6 side panel; 7 head-side side panel; 12 casing; 13 opening; 14 sealing member; 15 bendable portion; 16 operation portion; 17 switch; 18 LED; 19 vent; 21, 22 ion generating portion; 21a, 22a discharge electrode; 21b, 22b induction electrode ring; 31 hooded stroller; and 32 hood.

## Claims

1. A medical device for preventing or treating atopic dermatitis, comprising:
a covered space operable to cover a patient itself or an affected area;
an ion generating element operable to fill the covered space with positive ions composed of H⁺(H₂O)ₘ (m being any integer) and negative ions composed of O₂⁻(H₂O)ₙ (n being any integer) which are generated as a result of discharge in an atmosphere, said medical device configured such that said positive ions and said negative ions are brought into contact with a skin surface of the patient,
**characterised in that** the respective concentrations of said positive ions and said negative ions generated as a result of discharge are not less than 200000/cm³.

2. The device according to claim 1, wherein
the patient is a non-human mammal.

3. The device according to claim 1, wherein
the patient is a human.

4. The device according to claim 3, comprising:
a casing provided with a cavity for passage of an arm or a leg of a human therethrough; and
an ion generating element provided in the casing, for blowing ions into said cavity, wherein
said casing has an opening communicating with said cavity at each of opposing ends, and said opening has a sealing member for filling a gap between the arm or the leg of the human and said opening.

5. The device according to claim 4, wherein
said casing has a bendable portion between the openings at the respective opposing ends.

6. A hooded stroller, comprising:
the medical device of claim 1, wherein said covered space comprises a hood of a stroller.

## Patentansprüche

1. Medizinische Vorrichtung zum Vorbeugen oder Behandeln von atopischer Dermatitis, umfassend:
einen abgedeckten Raum, der wirksam ist, um einen Patienten selbst oder einen betroffenen Bereich abzudecken;
ein Ionen erzeugendes Element, das wirksam ist, um den abgedeckten Raum mit aus H⁺(H₂O)ₘ (wobei m eine beliebige ganze Zahl ist) bestehenden positiven Ionen und aus O₂⁻(H₂O)ₙ (wobei n eine beliebige ganze Zahl ist) bestehenden negativen Ionen, die als Folge von Entladungen in einer Atmosphäre erzeugt werden, zu füllen, wobei die medizinische Vorrichtung derart konfiguriert ist, dass die positiven Ionen und die negativen Ionen mit einer Hautoberfläche des Patienten in Kontakt gebracht werden,
**dadurch gekennzeichnet, dass** die jeweiligen Konzentrationen der als Folge von Entladungen erzeugten positiven Ionen und negativen Ionen nicht weniger als 200.000/cm³ betragen.

2. Vorrichtung nach Anspruch 1,
wobei es sich bei dem Patienten um ein nicht menschliches Säugetier handelt.

3. Vorrichtung nach Anspruch 1,
wobei es sich bei dem Patienten um einen Menschen handelt.

4. Vorrichtung nach Anspruch 3, die Folgendes umfasst:
ein Gehäuse, das mit einer Hohlraum zum Hindurchgelangen eines Arms oder eines Beins eines Menschen dadurch hindurch versehen ist; und
ein in dem Gehäuse bereitgestelltes Ionen erzeugendes Element zum Blasen von Ionen in den Hohlraum, wobei
das Gehäuse an jedem von gegenüberliegenden Enden eine Öffnung aufweist, die mit dem Hohlraum in Verbindung steht, und die Öffnung ein Dichtungselement zum Füllen eines Zwischenraums zwischen dem Arm oder dem Bein des Menschen und der Öffnung aufweist.

5. Vorrichtung nach Anspruch 4, wobei
das Gehäuse einen biegsamen Abschnitt zwischen den Öffnungen an den jeweiligen gegenüberliegenden Enden aufweist.

6. Kinderwagen mit Haube, der Folgendes umfasst:
die medizinische Vorrichtung nach Anspruch 1, wobei der abgedeckte Raum eine Haube eines Kinderwagens umfasst.

## Revendications

1. Dispositif médical destiné à prévenir ou à traiter la dermatite atopique, comprenant :
un espace couvert capable de fonctionner pour couvrir un patient même ou une zone affectée ;
un élément générateur d'ions capable de fonctionner pour remplir l'espace couvert avec des ions positifs composés de H⁺(H₂O)ₘ (m étant tout nombre entier) et des ions négatifs composés de O₂⁻(H₂O)ₙ (n étant tout nombre entier) qui sont générés suite à une décharge dans une atmosphère, ledit dispositif médical configuré de sorte que lesdits ions positifs et lesdits ions négatifs sont amenés en contact avec une surface cutanée du patient,
**caractérisé en ce que** des concentrations respectives desdits ions positifs et desdits ions négatifs générés suite à une décharge ne sont pas inférieures à 200 000/cm³.

2. Dispositif selon la revendication 1, dans lequel le patient est un mammifère non humain.

3. Dispositif selon la revendication 1, dans lequel le patient est un humain.

4. Dispositif selon la revendication 3, comprenant :
un boîtier pourvu d'une cavité pour le passage d'un bras ou d'une jambe d'un humain à travers celui-ci ; et
un élément générateur d'ions fourni dans le boîtier, destiné à souffler des ions jusque dans ladite cavité, dans lequel
ledit boîtier a une ouverture qui communique avec ladite cavité au niveau de chacune des extrémités opposées, et ladite ouverture a un élément d'étanchéité destiné à remplir un intervalle entre le bras ou la jambe de l'humain et ladite ouverture.

5. Dispositif selon la revendication 4, dans lequel ledit boîtier a une partie pliable entre les ouvertures au niveau des extrémités opposées respectives.

6. Poussette à capuchon, comprenant :
le dispositif médical selon la revendication 1, dans lequel ledit espace couvert comprend un capuchon d'une poussette.
